## Europäisches Patentamt

## European Patent Office

## Office européen des brevets

(19)

(11) Veröffentlichungsnummer: **0 162 935**
**B1**

(12) # EUROPÄISCHE PATENTSCHRIFT

(45) Veröffentlichungstag der Patentschrift:
**28.12.88**

(21) Anmeldenummer: **84105817.5**

(22) Anmeldetag: **22.05.84**

(51) Int. Cl.⁴: **B 05 B 7/04,** A 61 D 7/04,
A 61 M 15/00

(54) Aerosol-Generator zur Herstellung von Aerosoltropfen unter 5 mu (cm-6).

(43) Veröffentlichungstag der Anmeldung:
**04.12.85 Patentblatt 85/49**

(45) Bekanntmachung des Hinweises auf die Patenterteilung:
**28.12.88 Patentblatt 88/52**

(84) Benannte Vertragsstaaten:
**AT BE CH DE FR GB IT LI NL SE**

(56) Entgegenhaltungen:
**BE-A- 658 437**
**DE-C- 2 840 723**
**FR-A- 757 356**
**GB-A- 525 736**
**US-A- 3 379 373**

(73) Patentinhaber: **MEM Müszaki Intézet, HU-2101 Gödöllö (HU)**
Patentinhaber: **Allatorvostudományi Egyetem, Landler J.u.2, HU-1078 Budapest (HU)**
Patentinhaber: **Ipari Müszergyár, Iklad Pf. 2, H-2170 Aszod (HU)**

(72) Erfinder: **Sulok, János, Dipl.-Ing., Oszkó u. 11, XVIII Budapest (HU)**
Erfinder: **Csoma, Mihály, Dipl.-Ing., Kerepesi u. 80/a, H-1148 Budapest (HU)**
Erfinder: **Mátyás, László, Dipl.-Ing., Fó u. 34, H-3183 Karancskeszi (HU)**
Erfinder: **Tamási, Géza, Dr., Kagyló u. 4, H-1051 Budapest (HU)**
Erfinder: **Schulz, Péterné, Alvinci u. 32, H-1022 Budapest (HU)**
Erfinder: **Kiss, Kálmán, Dipl.-Ing., Korvin krt. 16, H-2100 Gödöllo (HU)**
Erfinder: **Jakus, László, Dipl.-Ing., István köz 3, H-2100 Gödöllo (HU)**

(74) Vertreter: **Eitle, Werner, Dipl.-Ing. et al, Hoffmann, Eitle & Partner Patentanwälte Arabellastrasse 4, D-8000 München 81 (DE)**

ACTORUM AG

**Beschreibung**

Gegenstand der Erfindung ist ein Aerosolgenerator zur Herstellung von Aerosoltropfen unter 5 μm. Der erfindungsgemässe Aerosolgenerator ist als ein pneumatischer Zerstäuber aufgebaut und weist eine Anstoss-Trennvorrichtung auf. Ein Aerosolgenerator der im Oberbegriff des Anspruchs angegebenen Art ist in der GB-A-525 736 beschrieben.

Die Erfindung kann vorteilhaft zur Behandlung von Tieren durch die Atmungsorgane mit Arzneien, ausserdem zur Desinfizierung von geschlossenen Räumen oder zu sonstigen Aerosolbehandlungen verwendet werden.

Wie bekannt, werden durch die in Viehzuchtwirtschaften auftretenden Epidemien enorme Schäden verursacht, wodurch Tiere massenhaft erkranken und zugrundegehen. Manche Epidemien werden durch Impfung oder durch Tränkungsbehandlung bekämpft. Diese Schutzmethoden weisen jedoch den Mangel auf, dass im Falle der Impfung jedes einzelne Tier separat behandelt werden muss. Eine solche Behandlung erfordert aber viel menschliche Arbeit, grossen Zeitaufwand und viel Medikamente. Bei der Tränkungsbehandlung wird eine bedeutende Menge von Arznei oder Vakzin benötigt. Bei diesen zwei bekannten Methoden – obwohl die Arbeitskraft und Materialaufwand bedeutend sind – kann ein Erfolg nur mit neunzig Prozent gerechnet werden.

Bekannt ist weiterhin, dass die spezifische Menge des Arznei- oder Vakzinbedarfes vermindert und die Produktivität der Arbeit vergrössert werden können, wenn die Medikamente, Vakzine oder sonstigen Heilmittel durch die Atmungsorgane, d.h. aerogen in das Innere des Tieres – oder des Menschen – eingeleitet werden. Dies geschieht derart, dass aus den Medikamenten oder Behandlungsmitteln ein Aerosol gebildet wird, das durch das Tier eingeatmet wird.

Diese Methode ist zwar an und für sich bekannt, ihre Realisierung wurde jedoch grundsätzlich durch zwei Faktoren verhindert: einerseits ist die Leistung der bekannten Aerosol-Generatoren klein (0,2–0,5 dm$^3$/Stunde); andererseits ist die Verteilung der durch die bekannten Aerosolgeneratoren gebildeten Tropfen heterogen, oder sie können zur Behandlung von Tieren mit einer ausreichenden Wirksamkeit nicht verwendet werden. Von den beiden Faktoren ist der letztere entscheidend, da in die Lunge nur die Aerosoltropfen unter 5 μm leicht einströmen und in dem Blutkreis ihre Wirkung ausüben. Aerosoltropfen im Bereich von 5–10 μm strömen mit einer kleineren Wahrscheinlichkeit in die Lunge ein und Tropfen über 10 μm bleiben in dem oberen Luftgang stecken und entleeren sich nutzlos aus dem Organ. Sie verursachen in manchen Fällen in den Luftgängen Schäden und können in der Lunge Erstickungserscheinungen hervorrufen.

In der beigelegten Figur wird gezeigt, wie die Aerosoltropfen – abhängig von ihrer Grösse – und in welchem Verhältnis anhaften. Es ist zu sehen, dass die Aerosoltropfen um 2 μm – im allgemeinen diejenigen im Bereich von 1–5 μm – in den unteren Luftgängen anhaften, in die Lunge einströmen, während die, die über 10 μm sind, nicht in die unteren Luftgänge einströmen.

Es ist ein Aerosolgenerator mit dem Namen «Pulsfog» bekannt. Es ist ein tragbarer, auf dem thermodynamischen Prinzip betriebener, jedoch geräuscherzeugender Apparat. Eine andere Einrichtung mit dem Namen «Pogger» (USA) weist eine Pneumatik auf und dient in erster Linie zur Desinfizierung von leeren Räumlichkeiten. Man versucht auch mit diesen Generatoren Aerosol-Immunisationsexperimente durchzuführen, ihre Wirksamkeit war aber schwächer als die der Tränkungsmethode.

Aus der DE-PS 2 840 723 ist ein mit Drallschaufel ausgestatteter Generator bekannt, bei dem auf der Antriebsmotorwelle eine mit Schaufelrad versehene Scheibe montiert ist. An diese Scheibe wird das grössere Ende eines trichterförmigen Saugrohres angeschlossen. Das Ende mit kleinerem Durchmesser taucht in die Flüssigkeit ein und transportiert diese durch eine Öffnung an die obere Fläche der Scheibe.

Wenn der Motor sich zu drehen beginnt, saugt der Generator durch einen zu diesem Zweck gebildeten Kanal Luft ein; die Luft strömt sich um den Motor drehend nach unten und erreicht die mit Schaufelrad versehene Scheibe. Die nach dem Fliehkraftprinzip zerstäubte Flüssigkeit wird durch die durch die Scheibe gelieferte Luft mitgerissen und liefert sie über Lenkkammer und Filter zu dem Ausgang hin. Durch diese Einrichtung werden Aerosoltropfen im Bereiche von 0,3–15 μm hergestellt. Da ihre Leistung klein ist, ist darüber hinaus die Abmessung der hergestellten Tropfen nicht für eine optimale aerogene Behandlung von Tieren oder Menschen geeignet.

Aus der US-PS 1 399 490 ist ein Aerosolgenerator bekannt, der nach dem Prinzip einer Fliehkraft-Scheibe betätigt wird. Diese Vorrichtung wird in erster Linie mit einem Luftreinigungsapparat zur Zerstäubung von Wasser verwendet. Aus der Fachliteratur ist bekannt, dass Zerstäuber dieser Art Tropfen von 50–80 μm herstellen und diese nicht für eine aerogene Behandlung geeignet sind.

Ein weiterer Aerosolgenerator ist aus der US-PS 4 116 387 bekannt, der als ein durch Hochdruckluft betätigter, mit Lenkkammer versehener Flüssigkeitszerstäuber zu betrachten ist, durch den Tropfen unter 0,2 μm hergestellt werden. Dieser ist aber ebenfalls nicht geeignet, die gewünschte Wirkung hervorzurufen.

Eine Weiterentwicklung der letztgenannten Lösung ist der Aerosolgenerator der US-PS 4 251 033. Nach analogem Prinzip betätigt werden auch hiermit Aerosoltropfen unter 0,1 μm hergestellt. Er ist also nicht geeignet für aerogene Behandlung.

Ein gemeinsamer Mangel der bekannten Aerosolgeneratoren besteht daher darin, dass ihre Leistung klein ist und sie ausserdem nicht geeignet sind zur Behandlung von Tieren grösserer Anzahl oder zur Desinfizierung von grösseren geschlossenen Räumen. Andererseits sind die hergestellten Tropfen entweder zu klein, oder können nicht innerhalb der nötigen Grenzen gehalten werden, wodurch eine wirksame Behandlung mit ihrer Hilfe nicht durchführbar ist.

Der Erfindung liegt die Aufgabe zugrunde, einen Aerosolgenerator zu schaffen, durch den die erwähnten Mängel eliminiert werden können. Der erfindungsgemässe Aerosolgenerator weist eine ausreichende Leistung auf. Die austretenden Aerosoltropfen befinden sich in dem für optimal betrachteten Bereich unter 5 μm mit deren Hilfe die Aerosolbehandlung von Tieren oder Menschen durchgeführt werden kann.

Der Erfindung liegt die Erkenntnis zugrunde, dass ein Aerosolgenerator – der nach dem pneumatischen Prinzip mit Drallschaufel als Zerstäuber betrieben wird – mit einer entsprechenden Anstoss-Trennvorrichtung versehen ist, der nur Tropfen unter 5 μm durch die Auslassöffnung des Generators hindurchlässt.

Der erfindungsgemässe Aerosolgenerator ist zur Herstellung von Aerosoltropfen unter 5 μm ausgestaltet und ist insbesondere geeignet zur Behandlung von Tieren durch deren Atmungsorgane. Er weist eine durch Drallschaufel betriebene Pneumatik und eine Anstoss-Trennvorrichtung auf.

Das Wesen des erfindungsgemässen Aerosolgenerators besteht darin, dass er einen zur Herstellung von Hochdruckluft dienenden Ventilator, einen Schleuderschaufel-Luftablenker, eine aus kegelstumpfförmigen oder aus kegelstumpfmantelförmigen Elementen bestehende ineinander verschiebbare Jalousie-Trennvorrichtung, sowie ein zum Rückführen der abgetrennten Aerosoltropfen dienendes Luftablenkergehäuse und gegebenenfalls einen als ein Maschinengerüst ausgebildeten Flüssigkeitsbehälter aufweist, während zwischen dem Flüssigkeitsbehälter und dem Zerstäuber eine zum Fördern der Flüssigkeit dienende Leitung angeordnet ist.

Nach einem vorteilhaften Ausfürungsbeispiel ist diese Leitung als ein mit Filter versehenes Rohr ausgebildet. Nach einem weiteren vorteilhaften Ausführungsbeispiel sind die wichtigsten Einheiten des Generators mit schnellverbindenden Elementen aneinandergekoppelt. Der Ventilatormotor des Generators wird vorteilhaft mit einem programmgesteuerten Stromkreis verbunden.

Die Erfindung wird anhand der beigelegten Zeichnungen näher erörtert.

Fig. 1 zeigt ein Diagramm, in dem das prozentuale Verhältnis der an den oberen und unteren Luftkanälen des Atmungsorganes anhaftenden Aerosolteilchen nach dem Tropfendurchmesser gezeigt wird;

Fig. 2 zeigt skizzenhaft den Aerosolgenerator und

Fig. 3 zeigt die am Ausgang des Generators erscheinende Tropfenkonzentration, abhängig von der Tropfengrösse.

Der erfindungsgemässe Aerosolgenerator ist als ein pneumatischer Schleuderschaufelzerstäuber ausgebildet, der ausserdem eine Anstoss-Trennvorrichtung aufweist.

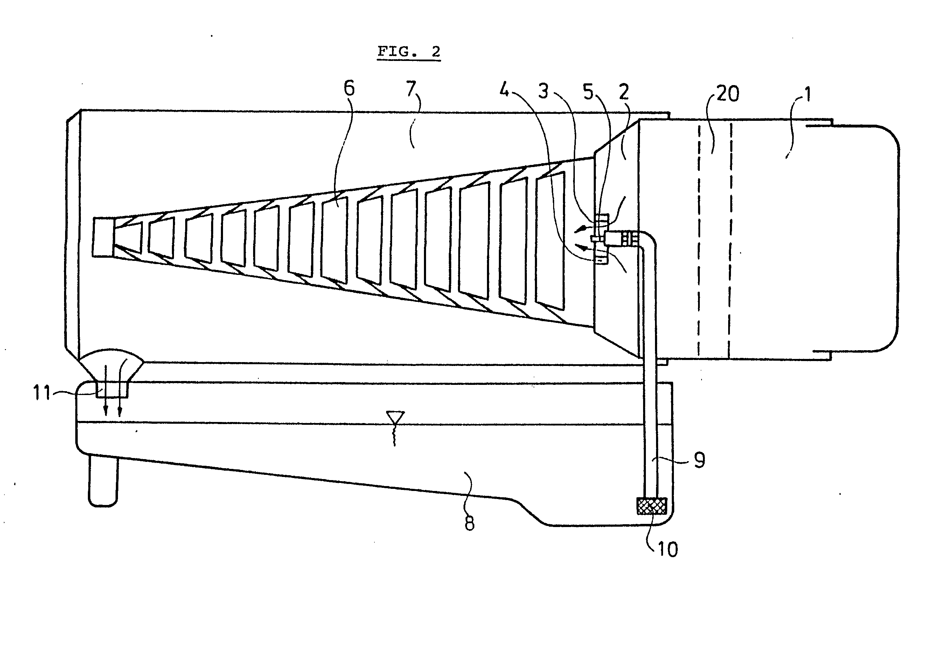

Der Aerosolgenerator (Fig. 2) weist einen mit Antriebsmotor 1 verbundenen Ventilator 20 auf, dessen Ausblasöffnung 3 an einem mit zur Drehachse konzentrischer Bohrung versehenen Gehäuse derart gebildet ist, dass die ausströmende Luft durch einen mit Schleuderschaufel versehenen Luftablenker 4 in Schleuderbewegung gebracht wird. In der Symmetrie-Linie des Luftablenkers 4 ist eine Spritzdüse 5 angeordnet. An das Gehäuse 2 des Hochdruckventilators 20 ist ein zylindrischer Luftablenker 7 angeschlossen. In diesem Luftablenker 7 ist die nach aussen verjüngte Jalousie-Trennvorrichtung 6 angeordnet, die aus kegelstumpfförmigen oder ineinander verschiebbaren, kegelstumpfförmigen Stücken besteht. Die Jalousie-Trennvorrichtung 6 ist an die Stirnwand des Ventilatorgehäuses 2 anmontiert. Der Jalousie-Abscheider 6 ist derart ausgebildet, dass er nur die Flüssigkeitstropfen unter 5 μm mit der durchströmenden Luft durchlässt. Die Tropfen grösserer Abmessung werden von der kegelstumpfförmigen Trennvorrichtung 6 in den zylindrischen Luftablenker 7 abgeschieden. Von dort gelangen sie durch die Öffnung 11 in den Flüssigkeitsbehälter 8 zurück.

Am Flüssigkeitsbehälter 8 ist eine weitere Öffnung vorgesehen, durch die eine den Flüssigkeitsbehälter 8 und den Zerstäuber verbindende Leitung 9 hindurchgeführt ist, die zur Zufuhr der Flüssigkeit zum Zerstäuber dient. Die Leitung 9 ist zweckdienlich ein Rohr, dessen in die Flüssigkeit getauchter Teil mit einem Filter 10 versehen ist. Der Luftablenker 4 mit Schleuderschaufel ist an der Ausblaseröffnung 3 des Ventilators angeordnet. Die Jalousie-Trennvorrichtung 6 ist in dem Luftablenker 7 derart angeordnet, dass sich ihr Querschnitt von der Zerstäubungsstelle ausgehend kontinuierlich verjüngt. Die Leitung 9 kann auch als ein flexibles Rohr ausgebildet sein, während der Flüssigkeitsbehälter 8 als ein mit einer Abtrennvorrichtung 6 versehenes Maschinengerüst ausgebildet sein kann.

Der erfindungsgemässe Aerosolgenerator funktioniert wie folgt:

Das zu zerstäubende Mittel (Medikament, Desinfiziermittel, usw.) wird durch die Öffnung 11 in den Flüssigkeitsbehälter 8 gegossen. Hierauf wird die mit der Jalousie-Abtrennvorrichtung versehene Generatoreinheit an den Behälter angeschlossen und befestigt.

Die wesentlichen Verbindungselemente, wie z. B. die den Antriebsmotor 1, den Hochdruckventilator 20 sowie die Jalousie-Abtrennvorrichtung 6 verbindenden Elemente sind als Schnellverbindungen ausgebildet, weil sie auf diese Weise nach dem Gebrauch leichter und einfacher gesäubert werden können. Es sollten so viele Generatoren verwendet werden, wie gemeinsam zu der Behandlung des gegebenen Raumes auf einmal nötig sind.

Im Falle der Anwendung von mehreren Generatoren werden die Speiseleitungen parallel geschaltet, damit sie gleichzeitig betrieben werden können. Die ganze Einheit kann derart in Funktion gesetzt werden, dass die einzelnen Funktionselemente nach dem gesperrten Zustand des Raumes sich automatisch einschalten. Ein grosser Vorteil des erfindungsgemässen Aerosolgenerators besteht darin, dass seine Betätigung von aussen her gesteuert werden kann. Im Inneren des zu behandelnden Raumes soll sich kein Bedienungspersonal aufhalten, und die Einheit lässt sich zu gleicher Zeit von aussen kontrollieren.

Die Anlage kann auch derart betrieben werden, dass die Antriebsmotore der Generatoren mit einem programmierbaren Steuerstromkreis versehen sind. Wenn der Antriebsmotor 1 des Generators angefahren ist, kann die durch den Hochdruckventilator 20 gelieferte Luft aus dem Gehäuse 2 des Ventilators nur durch die mit dem Schleuderschaufel-Luftablenker versehene Ausblaseöffnung 3 ausströmen, während der Druck der gelieferten Luft sich vermindert, wodurch das Absaugen des zu Aerosol zu zerstäubenden Mittels (Flüssigkeit) durch die Öffnung des Zerstäubers 5 erfolgt.

Die ausströmende Luft, die das zerstäubte Mittel enthält, tritt aus dem Luftablenker 7 erst dann in den zu behandelnden Raum ein, wenn sie vorher durch die kegelstumpfförmige, am Ende geschlossene Jalousie-Abtrennvorrichtung 6 kleinen Querschnittes hindurchgeströmt ist, wodurch die Luft zu einer mehrfachen Richtungsänderung und zu einer kontinuierlichen Turbulenz gezwungen wird. Unterdessen schlagen sich infolge der Kegelstumpfform und des sich verjüngenden Querschnittes die Tropfen, die grösser als 5 μm sind, nieder und gelangen durch die Öffnung 11 in den Flüssigkeitsbehälter 8 zurück.

Die in der ausströmenden Luft zurückgebliebenen Aerosoltropfen, die kleiner als 5 μm sind, verteilen sich in den zu behandelnden Raum. Eine gewünschte Aerosolkonzentration wird durch die Betriebsdauer sowie durch die Auswahl der Maschinenanzahl gesichert. Die Betriebsdauer ist abhängig von der Leistung der Aerosolgeneratoren, so dass die Betätigung der ganzen Einrichtung automatisiert werden kann.

Infolge der Leistung des erfindungsgemässen Aerosolgenerators kann die Behandlung von sehr grossen Räumen gleichzeitig durchgeführt werden und zwar derart, dass die entsprechende Tropfenverteilung gesichert wird.

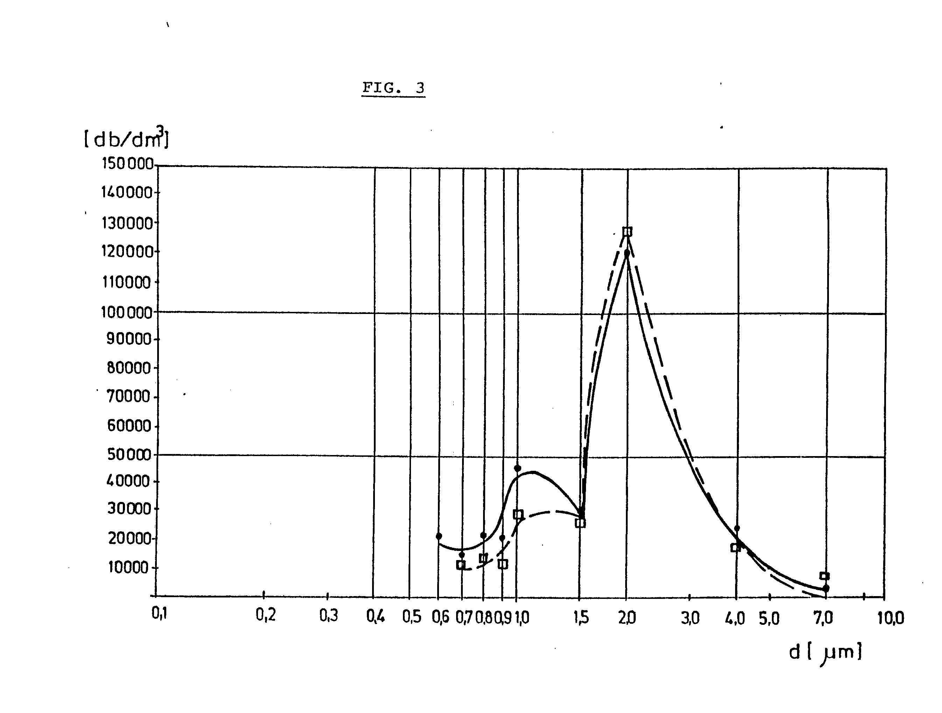

In Fig. 3 ist ein Diagramm gezeigt, in welchem die Tropfenkonzentration der durch den erfindungsgemässen Aerosolgenerator ausgeschiedenen Tropfen je nach der Tropfengrösse veranschaulicht ist. In der Figur sieht man eine Konzentrationsverteilung mit der verdünnenden Luft zusammen, weil das zur Verfügung stehende Messgerät einen beschränkten Messbereich aufweist. Es ist zu sehen, dass der grösste Teil der Tropfen eine Abmessung von 1,4 bis 4 μm aufweist, wobei ihr Maximum in den Bereich von 2 μm fällt.

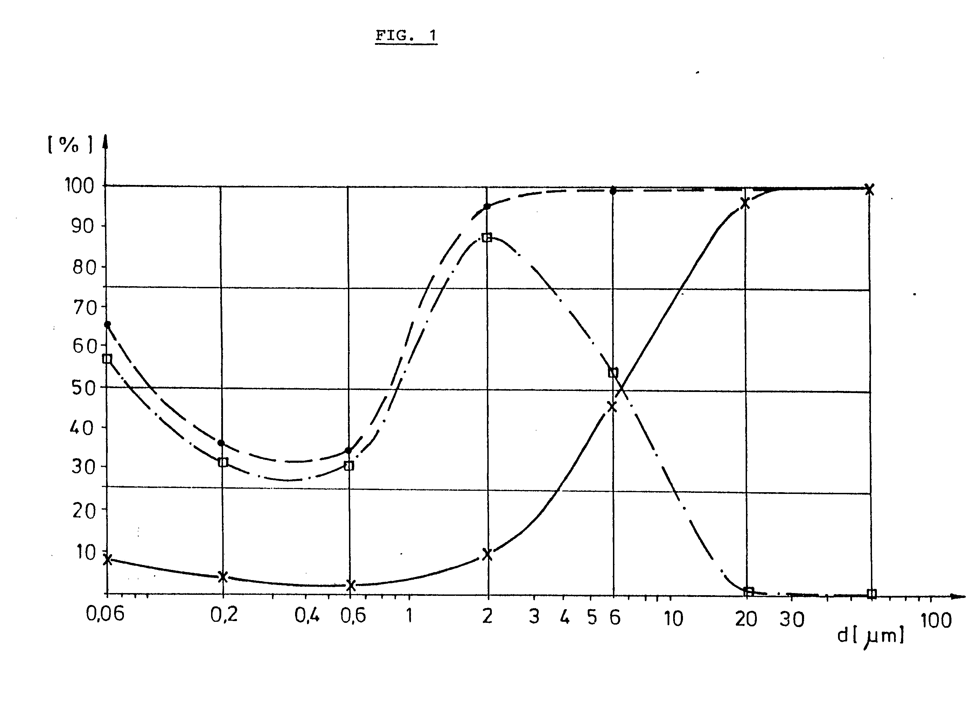

Zurückkommend auf Fig. 1 sieht man, dass eine optimale Behandlung durch die unteren Luftgänge gesichert ist.

Mit Hilfe der Erfindung kann die Behandlung mit einer verhältnismässig kleinen Menge von Behandlungsmitteln optimal und rasch durchgeführt werden, was bei der Viehzucht sowohl bei preventiver Behandlung als auch bei einer Seuchenbehandlung bedeutsam ist.

**Patentansprüche**

1. Aerosolgenerator zur Herstellung von Aerosoltropfen zweckmässig unter 5 μm, insbesondere zur Behandlung der Atmungswege von Tieren, wobei der Aerosolgenerator als pneumatischer Ansaugzerstäuber ausgebildet ist, mit einem der Erzeugung von Druckluft dienenden Ventilator, einer kegelstumpf- oder kegelstumpfmantelförmigen Abtrennvorrichtung, einem zum Rückführen der abgeschiedenen Aerosoltropfen dienenden Luftablenkergehäuse und gegebenenfalls einem Flüssigkeitsbehälter, der über eine Flüssigkeitsförderleitung mit dem Zerstäuber verbunden ist, dadurch gekennzeichnet, dass ein mit einer Schleuderschaufel versehener Luftablenker (4) vorgesehen ist, dass die Abtrennvorrichtung (6) in Form einer Jalousie aus kegelstumpfförmigen oder kegelstumpfmantelförmigen Elementen besteht, die ineinander verschiebbar sind.

2. Aerosolgenerator nach Anspruch 1, dadurch gekennzeichnet, dass der Flüssigkeitsbehälter (8) als Maschinengerüst dient.

**Claims**

1. Aerosol generator for the production of aerosol droplets expediently below 5 μm, particularly for the treatment of the respiratory pathways of animals, wherein the aerosol generator is formed as a pneumatic suction atomiser, with a fan serving for the production of pressurised air, a separating device for frusto-conical shape or having a frusto-conically shaped outer surface, an air deflector housing serving for returning the separated aerosol droplets and optically a liquid container connected via a liquid conveying duct with the atomiser, characterised in that there is provided an air deflector (4) equipped with a centrifugal blade, that the separating device (6) consists of louvring made up of elements of frusto-conical shape or having a frusto-conically shaped outer surface which are displaceable into each other.

2. Aerosol generator according to claim 1, characterised in that the liquid container (8) serves as a machine frame.

**Revendications**

1. Générator d'aérosol pour l'obtention de gouttes d'aérosol avantageusement inférieures à 5 μm, notamment pour le traitement des voies respiratoires des animaux, dans lequel le générateur d'aérosol est réalisé sous forme d'un pulvérisateur pneumatique par aspiration comprenant un ventilateur servant à produire de l'air comprimé, un dispositif de séparation en forme de tronc de cône ou d'enveloppe de tronc de cône, un carter de déviation de l'air servant à la recirculation des gouttes d'aérosol séparées et, le cas échéant, un récipient de liquide, qui est relié par une conduite d'alimentation de liquide au pulvérisateur, caractérisé en ce qu'il comporte un dispositif de déviation de l'air (4) pourvu d'une roue centrifuge à palettes, en ce que le dispositif de séparation (6), en forme de jalousie, est composé d'éléments en forme de tronc de cône ou d'enveloppe, de tronc de cône et susceptibles d'être déplacés l'un dans l'autre (téléscopiquement).

2. Générateur d'aérosol selon la revendication 1, caractérisé en ce que le récipient de liquide (8) sert de support mécanique pour le générateur.

FIG. 1

FIG. 2

EP 0 162 935 B1

FIG. 3